# EUROPÄISCHE PATENTANMELDUNG

(11) **EP 2 189 124 A1**
(43) Veröffentlichungstag der Anmeldung: **26.05.2010**
(21) Anmeldenummer: 08020138.7
(22) Anmeldetag: 19.11.2008
(51) Int. Cl.: A61B 17/70

(54) **Dornfortsatz-Implantat**

(71) Anmelder: Röbling, Christian, 79104 Freiburg (DE); Ackermann, Rolf, 78604 Rietheim-Weilheim (DE)
(72) Erfinder: Röbling, Christian, 79104 Freiburg (DE); Ackermann, Rolf, 78604 Rietheim-Weilheim (DE)
(74) Vertreter: Patentanwälte Westphal, Mussgnug & Partner

(57) **Zusammenfassung**

Ein Dornfortsatz-Implantat ist mit zur medianen Sagittalebene des Patienten im Wesentlichen senkrechter Längsachse zwischen einem oberen und einem anschließenden unteren Dornfortsatz positionierbar. Das Implantat weist als Abstandhalter obere Stützmittel (10.1) und untere Stützmittel (10.2) auf, die jeweils mit einem vertieften Sattel (12) an dem oberen bzw. unteren Dornfortsatz zur Anlage kommen. Die Stützmittel (10.1, 10.2) sind mittels Spannmitteln (16, 18) aufspreizbar, welche zwischen den Stützmittel (10.1, 10.2) in Längsrichtung angeordnet sind.

## Beschreibung

Die Erfindung betrifft ein Dornfortsatz-Implantat gemäß dem Oberbegriff des Patentanspruchs 1.

Eine Degeneration der Wirbelsäule, die insbesondere bei zunehmenden Lebensalter auftritt, führt häufig zu einer lumbalen Spinalkanalstenose. Hierbei handelt es sich um eine Verengung des Spinalkanals und der Zwischenwirbellöcher (foramina intervertebralia) im Allgemeinen als Folge einer Bandscheibendegeneration. Typische Symptome sind Nervenschmerzen im Rücken und in den Beinen und in schwereren Fällen Lähmungserscheinungen in den Beinen.

In einfachen Fällen kann eine Behandlung mit Medikamenten und Krankengymnastik erfolgreich sein. In schwereren Fällen wird eine Dekompression der Nervenstrukturen durch eine Distraktion der Wirbel bewirkt. Dabei können für eine Langzeittherapie Implantate zwischen die Dornfortsätze der betroffenen Wirbel eingesetzt werden. Gemeinsam ist diesen Dornfortsatz-Implantaten ein Abstandhalter, der zwischen den Dornfortsatz des oberen, kranialen Wirbels und den Dornfortsatz des anschließenden unteren kaudalen Wirbels eingesetzt wird. Der Abstandhalter wird dabei lateral zwischen die Dornfortsätze eingeführt, wobei seine Längsachse im Wesentlichen senkrecht zur medianen Sagittalebene, d. h. zur Körpermittelebene angeordnet ist.

Aus der US 5,860,977 ist ein Dornfortsatz-Implantat bekannt, bei welchem der Abstandhalter im Wesentlichen ein zylindrischer Körper ist, der zwischen die Dornfortsätze eingesetzt wird und gegen eine laterale Verschiebung in seiner Längsachse durch an seinen beiden lateralen Enden angeordnete Flügel gesichert wird. Ein unilaterales Einsetzen des Implantats ist dabei nicht möglich, da der Abstandhalter mit einem Flügel von der einen Seite eingesetzt wird und anschließend der zweite Flügel auf der anderen Seite des Dornfortsatzes aufgesetzt werden muss.

Eine unilateral einsetzbares Implantat wird von der Firma Kyphon unter der Bezeichnung "Aperius" vertrieben. Dieses Implantat weist einen rohrförmigen Abstandhalter mit zwei beabstandeten Verdünnungszonen auf. Der Abstandhalter wird unilateral zwischen die Dornfortsätze eingeführt und dann in Längsrichtung gestaucht, so dass die Verdünnungszonen zu radial abstehenden Flügeln verformt werden, die den Abstandhalter beiderseits der Dornfortsätze positioniert halten.

Diesen Dornfortsatz-Implantaten ist gemeinsam, dass der Abstandhalter einen vorgegebenen Durchmesser aufweist. Je nach den anatomischen Gegebenheiten müssen daher Abstandhalter mit unterschiedlichen Durchmessern eingesetzt werden. Zum einen muss daher in einem ersten operativen Schritt der interspinale Raum gemessen werden, um den passenden Abstandhalter auswählen zu können. Außerdem muss der Abstandhalter in unterschiedlichen Größen zur Verfügung stehen.

In der EP 1 330 987 B1 ist ein Dornfortsatz-Implantat beschrieben, bei welchem als Abstandhalter ein geschlossenes Federband verwendet wird, welches im Wesentlichen die Form einer zur Sagittalebene symmetrischen Acht hat. Das Implantat wird mit Befestigungslaschen an den jeweiligen Dornfortsätzen befestigt. Das Implantat fixiert somit die Dornfortsätze der aufeinanderfolgenden Wirbel gegenseitig, so dass eine Flexion der Wirbelsäule nur noch in den engen Grenzen der elastischen Verformbarkeit des Implantats möglich ist. Auch hier muss das Implantat jeweils in einer den anatomischen Voraussetzungen angepassten Größe gewählt werden.

Der Erfindung liegt die Aufgabe zu Grunde, ein Dornfortsatz-Implantat zur Verfügung zu stellen, welches einfach implantiert werden kann und für unterschiedliche anatomische Verhältnisse verwendbar ist.

Diese Aufgabe wird erfindungsgemäß gelöst durch ein Dornfortsatz-Implantat mit den Merkmalen des Patentanspruchs 1.

Vorteilhafte Ausführungen der Erfindung sind in den Unteransprüchen angegeben.

Der wesentliche Gedanke der Erfindung besteht darin, als Abstandhalter, der zwischen die Dornfortsätze eingesetzt wird, nicht einen Körper mit vorgegebenem festem Durchmesser zu verwenden, sondern einen Abstandhalter, der zwei Stützmittel aufweist, die auseinandergespreizt werden können. In einer Einführstellung liegen die Stützmittel mit einem geringen Abstand aneinander an, so dass der Abstandhalter nur einen geringen Durchmesser aufweist. Nach dem Einführen zwischen die Dornfortsätze werden die Stützmittel auseinandergespreizt, so dass sich ihr Abstand vergrößert. Die oberen Stützmittel legen sich dadurch an den oberen kranialen Dornfortsatz an und die unteren Stützmittel an den anschließenden unteren kaudalen Dornfortsatz. Dadurch kann sich der Abstandhalter den jeweiligen anatomischen Verhältnissen anpassen und es sind nicht unterschiedliche Abstandhalter für unterschiedliche Abmessungen des Interspinalraumes erforderlich.

Zum Auseinanderspreizen der Stützmittel dienen dabei Spannmittel. Diese sind zwischen den Stützmitteln in der Längsachse des Implantat, d.h. in der zur medianen Sagittalebene senkrechten Achse angeordnet und greifen an den beiden lateralen Enden der Stützmittel an. Dadurch ist ein unilaterales Einführen des Implantats möglich. Das Implantat kann durch eine perkutane Inzision unilateral zwischen die Dornfortsätze eingeführt werden und wird dann durch die unilaterale Betätigung der Spannmittel aufgespreizt und zwischen den Dornfortsätzen positioniert und fixiert. Die Stützmittel weisen jeweils einen vertieften Sattel auf, der eine Aufnahme für den jeweiligen Dornfortsatz bildet. Wenn die Stützmittel im aufgespreizten Zustand an dem jeweiligen Dornfortsatz anliegen, so legen sie sich mit diesem Sattel über einen Umfangsbereich am Umfang des Dornfortsatzes an, wodurch die Stützmittel und damit das gesamte Implantat formschlüssig gegen eine laterale Verschiebung, d. h. gegen eine Verschiebung in der zur Sagittalebene senkrechten Längsachse des Implantats gesichert sind. Das Implantat benötigt daher keine zusätzlichen Flügel oder sonstigen Maßnahmen zur Sicherung gegen eine laterale Verschiebung in Bezug auf die Dornfortsätze. Dies vereinfacht die Konstruktion und erleichtert insbesondere die Operationstechnik beim Einsetzen des Implantats.

In einer Ausführung sind die Stützmittel als Federblätter ausgebildet, deren laterale Enden durch die Spannmittel zusammengezogen werden, so dass die Federblätter nach oben bzw. nach unten konvex ausgewölbt und auseinandergespreizt werden.

In einer anderen Ausführung sind die Stützmittel als Stützplatten ausgebildet, die bei Betätigung der Spannmittel aus ihrer Einführstellung nach oben bzw. unten abgeschwenkt werden, so dass sich ihr Abstand vergrößert. In einer einfachen Ausführung können dabei an den beiden lateralen Enden jeweils eine obere und eine untere Stützplatte angelenkt sein, deren gegeneinander gerichtete freien Enden von der Längsachse nach oben bzw. nach unten abgeschwenkt werden.

Die Spannmittel sind in einer einfach zu handhabenden Ausführung teleskopisch ausgebildet. Durch ein teleskopisches Zusammenschieben können die lateralen Enden aufeinanderzu bewegt werden, um die Stützmittel aufzuspreizen. Das teleskopische Zusammenschieben kann dabei mittels eines selbsthemmenden Gewindes unilateral erfolgen. Die Spannmittel können auch frei teleskopisch ineinanderschiebbar ausgebildet sein, wobei die beiden Teleskopteile in der jeweiligen Spreizstellung gegen den Gegendruck der Dornfortsätze einrasten. In einer Weiterbildung der Erfindung können die Teleskopteile durch eine Nachstellkraft weiter ineinander geschoben werden, um die Stützmittel weiter aufzuspreizen, um das Implantat, z. B. bei Knochenresorption, dem sich vergrößernden Abstand der Dornfortsätze anzupassen.

Im Folgenden wird die Erfindung anhand von in der Zeichnung dargestellten Ausführungsbeispielen näher erläutert. Es zeigen
- Fig. 1: eine Seitenansicht einer ersten Ausführung des Dornfortsatz-Implantats in der Einführstellung,
- Fig. 2: eine perspektivische Ansicht des Implantats in der Spreizstellung,
- Fig. 3: eine weitere perspektivische Ansicht dieses Implantats,
- Fig. 4: einen Längsschnitt der ersten Ausführung des Implantats,
- Fig. 5: eine zweite Ausführung des Dornfortsatz-Implantas in der Einführstellung,
- Fig. 6: die zweite Ausführung in der Spreizstellung,
- Fig. 7: eine perspektivische Ansicht der zweiten Ausführung, wobei die Stützplatten weggelassen sind,
- Fig. 8: eine Teilansicht zu Fig. 7,
- Fig. 9: eine weitere Teilansicht, bei welcher eine Stützplatte zur Verdeutlichung gezeigt ist und
- Fig. 10: eine Detailansicht einer Ausführung der Spannmittel.

Das erfindungsgemäße Dornfortsatz-Implantat dient dazu, die Dornfortsätze (Processus spinosus) zweier aufeinanderfolgender Wirbel, in den meisten Fällen zwei Lendenwirbel vertikal beabstandet zu halten. Hierzu wird das Implantat zwischen die Dornfortsätze von lateral nach medial eingeführt und so positioniert, dass die Längsachse des Implantats im Wesentlichen senkrecht zur medianen Sagittalebene des Patienten, d. h. zur Körpermittelebene angeordnet ist. Zur einfacheren Beschreibung beziehen sich die nachfolgenden Angaben auf die implantierte Lage des Implantats. Mit "oben" bzw. "unten" werden dementsprechend die kranial bzw. kaudal gerichteten Seiten des implantierten Implantats bezeichnet. Mit "lateral" und "rechts" bzw. "links" werden dementsprechend die bei implantiertem Implantat jeweils seitlich von der medianen Sagittalebene liegenden Bereiche bezeichnet.

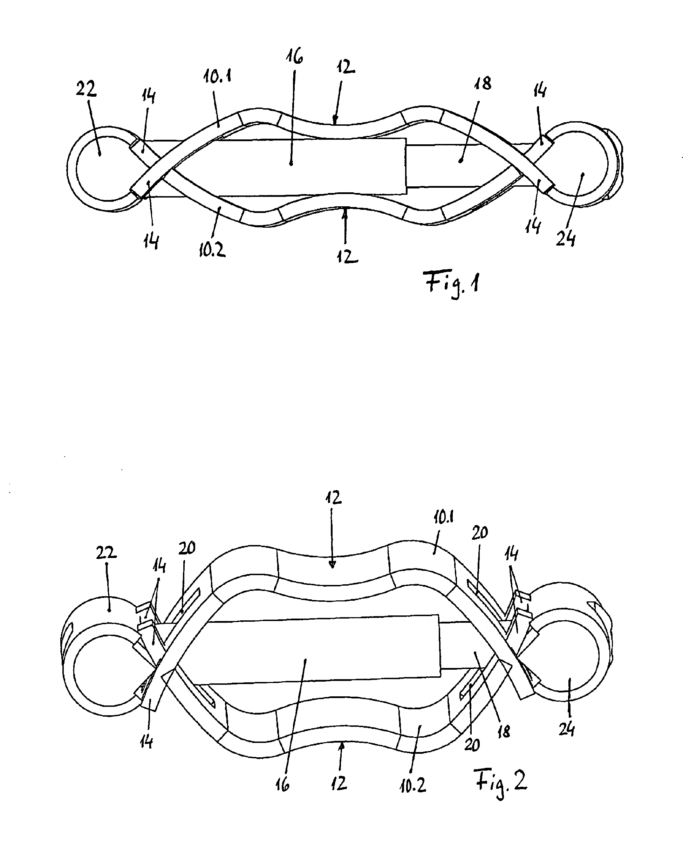

In den Figuren 1 bis 4 ist eine erste Ausführung des Dornfortsatz-Implantats gezeigt. Das Implantat weist obere und untere Stützmittel auf, die jeweils als Federblatt 10 ausgebildet sind. Das obere Federblatt 10.1 und das untere Federblatt 10.2 sind dabei in ihrer Form identische Teile, die jedoch um die Längsmittelachse des Implantats um 180° gegeneinander verdreht angeordnet sind. Die Federblätter 10 haben die Form eines langgestreckten flachen Bandes, welches formelastisch ist, d.h. seine Form zwar beibehält, jedoch gegen eine relativ starke elastische Rückstellkraft verformbar ist. Die Federblätter 10 sind hierzu aus einem geeigneten biokompatiblen Werkstoff hergestellt, z.B. aus einem Metall, insbesondere Titanlegierung, oder aus einem geeigneten Kunststoff, z.B. PEEK. Die Federblätter 10 weisen beispielsweise eine Länge von 15 bis 30 mm, vorzugsweise von 20 bis 25 mm, eine Breite von 4 bis 8 mm und eine Stärke von 0,8 bis 1,2 mm auf.

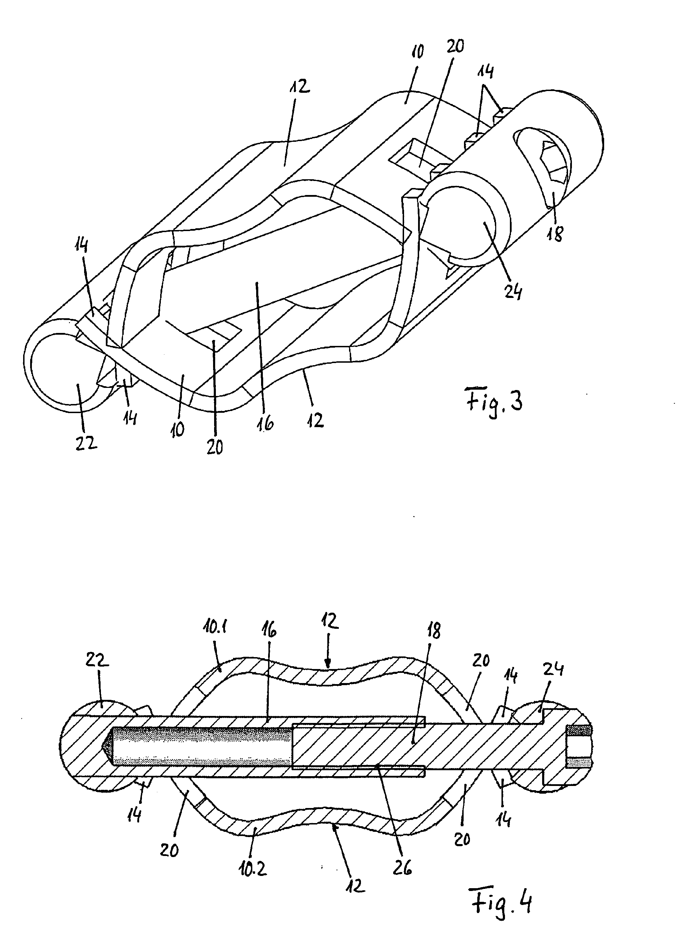

Die Federblätter 10 sind in ihrer Längsrichtung als flacher Bogen nach oben bzw. nach unten konvex ausgewölbt. Im mittleren Bereich ihrer Längserstreckung weisen die Federblätter 10 dabei eine konkave entgegengesetzt gewölbte Vertiefung auf, die einen Sattel 12 bildet. An ihren beiden lateralen Endkanten weisen die Federblätter 10 Einschnitte auf, so dass axial vorspringende Zähne 14 gebildet werden. Wenn die beiden Federblätter 10.1 und 10.2 um 180° gegeneinander verdreht zusammengefügt werden, greifen die Zähne 14 der jeweiligen lateralen Enden der beiden Federblätter 10.1 und 10.2 verschränkt ineinander, so dass sich die lateralen Enden der beiden Federblätter 10.1 und 10.2 überkreuzen, wie dies in den Figuren 2 und 3 zu sehen ist.

Zwischen die beiden Federblätter 10.1 und 10.2 sind in der Mittellängsachse des Implantats Spannmittel eingesetzt. Die Spannmittel bestehen aus einem Außenrohr 16 und einem teleskopisch in das Außenrohr 16 eingreifenden Innenbolzen 18. Das Außenrohr 16 und der Innenbolzen 18 greifen jeweils durch mittige axiale Einschnitte 20 der lateralen Endkanten der Federblätter 10. An den einander entgegengesetzten lateralen Enden des Außenrohres 16 und des Innenbolzens 18 ist jeweils ein Endstück 22 bzw. 24 angeordnet. Die Endstücke 22 und 24 haben die Form einer Rolle, deren Achse senkrecht zur Achse des Außenrohres 16 bzw. des Innenbolzens 18 verläuft. Die Endstücke 22 und 24 liegen jeweils lateral von außen in der Gabelung der sich überkreuzenden und verschränkten lateralen Enden der Federblätter 10.1 und 10.2.

In einer Einführstellung des Implantats, wie sie in Figur 1 gezeigt ist, sind das Außenrohr 16 und der Innenbolzen 18 mit den Endstücken 22 bzw. 24 in der Mittelachse des Implantats lateral nach außen auseinandergefahren. Die Federblätter 10.1 und 10.2 sind dadurch auf Grund ihrer elastischen Formstabilität flach gestreckt, so dass sie mit den einander zugewandten Innenflächen der Sattel 12 an den Spannmitteln, d.h. insbesondere an dem Außenrohr 16 anliegen. Das gesamte Implantat weist in dieser Einführstellung nur eine Höhe von etwa 5 bis 7 mm auf. In dieser Einführstellung wird das Implantat zwischen die Dornfortsätze zweier aufeinanderfolgender Wirbel eingesetzt. Hierzu wird bei entsprechender Lagerung des Patienten eine perkutane Inzision unilateral und parallel zur Mittelebene des Patienten vorgenommen und der Interspinalraum zwischen den Dornfortsätzen wird frei präpariert. Mittels eines geeigneten Einführinstruments wird das Implantat von lateral nach medial zwischen die Dornfortsätze eingeführt und im Interspinalraum möglichst nahe am Wirbelbogen positioniert. Das Implantat wird dabei so positioniert, dass die Mittelachse, d.h. die Achse der Spannmittel, nämlich des Außenrohres 16 und des Innenbolzens 18 im Wesentlichen senkrecht zur medianen Sagittalebene verläuft. Das obere Federblatt 10.1 ist mit seinem Sattel 12 gegen den Dornfortsatz des oberen Wirbels und das untere Federblatt 10.2 mit seinem Sattel 12 gegen den Dornfortsatz des unteren Wirbels gerichtet.

Dann werden die Spannmittel betätigt, indem der Innenbolzen 18 und das Außenrohr 16 koaxial ineinanderbewegt werden und dadurch die Endstücke 22 und 24 in lateraler Richtung gegeneinandergezogen werden. Durch die sich aufeinanderzubewegenden Endstücke 22 und 24 werden die verschränkten lateralen Enden der Federblätter 10.1 und 10.2 gegeneinandergezogen, so dass sich die Federblätter 10.1 und 10.2 konvex nach oben bzw. nach unten wölben und in vertikaler Richtung auseinandergespreizt werden. Diese Spreizstellung ist in Figur 2 gezeigt. Durch das Aufspreizen der Federblätter 10.1 und 10.2 werden diese gegen die jeweiligen Dornfortsätze gedrückt, um diese in der gewünschten Weise zu distrahieren. Da die Dornfortsätze dabei jeweils in dem Sattel 12 des oberen bzw. des unteren Federblattes 10 liegen, ist das Implantat gegen eine Verschiebung in lateraler Richtung im Wesentlichen formschlüssig gesichert. In der medialen Sagittalebene, d.h. in der Ebene senkrecht zu den Spannmitteln ist das Implantat durch die anatomische Konkavität der Dornfortsätze gesichert.

Das Spannen der Spannmittel kann in unterschiedlicher Weise realisiert werden. In einer Ausführung, wie sie in Figur 4 gezeigt ist, greift der Innenbolzen 18 mit einem Gewinde 26 in ein Innengewinde des Außenrohres 16. Das Gewinde 26 ist dabei selbsthemmend ausgebildet. Das Endstück 22 kann dabei fest mit dem Außenrohr 16 verbunden sein, während der Innenbolzen 18 das Endstück 24 frei drehbar durchsetzt. Der Innenbolzen 18 stützt sich axial mit einem Schraubenkopf an dem Endstück 24 ab und ist mittels dieses Schraubenkopfes von der lateralen Endseite her verdrehbar, um die Spannmittel zu spannen. Die Selbsthemmung des Gewindes 26 gewährleistet dabei, dass das implantierte Implantat in der gespannten und auseinandergespreizten Stellung verbleibt. Ist eine operative Entfernung des Implantats notwendig, so kann der Innenbolzen 18 wieder aus dem Außenrohr 16 heraugedreht werden, so dass das Implantat wieder in die Einführstellung gelangt. Weiter kann das Implantat in einem minimalinvasiven Eingriff mittels des Schraubenkopfes nachgespannt werden, wenn sich der Abstand der Dornfortsätze, z. B. in Folge von Knochenschwund, vergrößert.

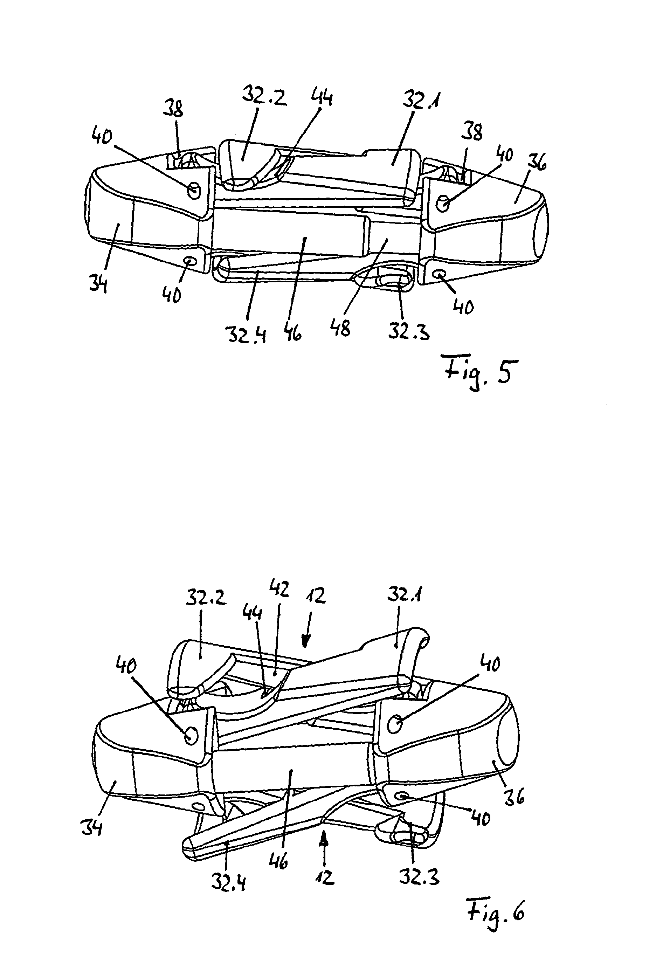

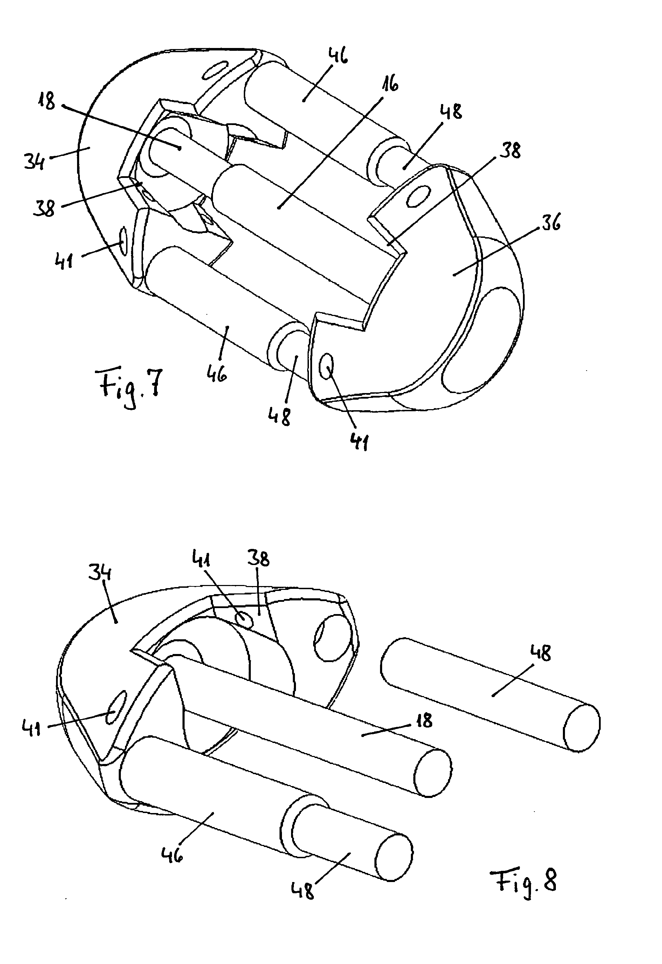

In den Figuren 5 bis 9 ist eine zweite Ausführung des Dornfortsatz-Implantats dargestellt.

In dieser Ausführung sind die Stützmittel als Stützplatten 32 ausgebildet. Es sind an der Oberseite eine linke Stützplatte 32.1 und eine rechts Stützplatte 32.2 und entsprechend an der Unterseite eine linke Stützplatte 32.3 und eine rechte Stützplatte 32.4 vorgesehen. Die Stützplatten 32.1, 32.2, 32.3 und 32.4 weisen alle eine identische Form auf. Die Stützplatten 32 bestehen aus einem geeigneten biokompatiblen formstabilen Material, beispielsweise aus Metall, insbesondere einer Titanlegierung oder vorzugsweise aus einem geeigneten Kunststoff, insbesondere PEEK. Die identische Form der Stützplatten 32.1, 32.2, 32.3 und 32.4 ist in Bezug auf die Herstellungskosten vorteilhaft.

Die Stützplatten 32 weisen die Form einer in Längsrichtung langgestreckten Platte auf, deren Länge etwa 15 bis 30 mm und deren Breite etwa 4 bis 8 mm beträgt. Die Materialstärke beträgt etwa 1 bis 2 mm. Die Stützplatten 32 sind jeweils an lateralen Endstücken 34 bzw. 36 angelenkt, wobei die Endstücke 34 und 36 über Spannmittel in lateraler Richtung gegeneinander gezogen werden können. Die Endstücke 34 und 36 haben dabei im Wesentlichen dieselbe Form eines Blockes.

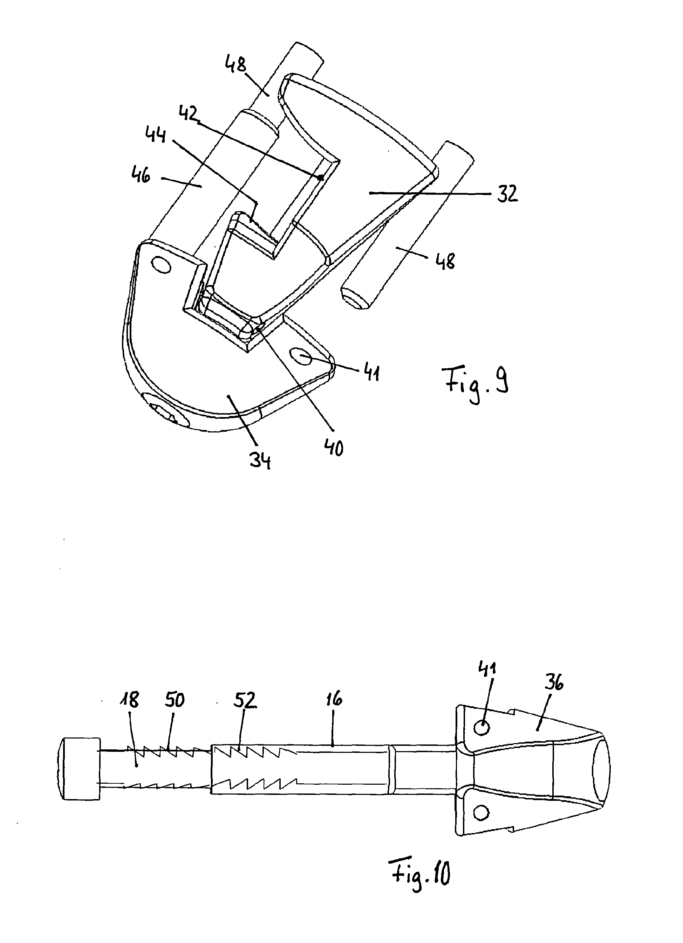

Zur schwenkbaren Lagerung der Stützplatten 32 an den Endstücken 34 bzw. 36 weisen diese Endstücke 34, 36 an ihren einander zugewandten Seiten jeweils an der Oberseite und an der Unterseite Aussparungen 38 auf. In diese Aussparungen 38 ist jeweils ein Ende einer Stützplatte 32 eingesetzt und um eine Schwenkachse 40 schwenkbar gelagert, die die Aussparung 38 jeweils parallel zur Oberseite bzw. Unterseite des Implantats und senkrecht zu dessen Mittelachse durchsetzt und in Bohrungen 41 eingesetzt ist. Auf diese Weise ist die obere linke Stützplatte 32.1 mit ihrem linken Ende schwenkbar in der oberen Aussparung 38 des linken Endstückes 34 gelagert, wobei das andere freie Ende dieser Stützplatte 32.1 nach rechts gerichtet ist. Entsprechend ist die rechte obere Stützplatte 32.2 in einer oberen Aussparung 38 des rechten Endstückes 36, die linke untere Stützplatte 32.3 in einer unteren Aussparung 38 des linken Endstückes 34 und die rechte untere Stützplatte 32.4 in einer unteren Aussparung 38 des rechten Endstückes 36 schwenkbar gelagert. Die Stützplatten 32 weisen an ihrem jeweiligen in der Aussparung 38 gelagerten Ende eine die Aussparung 38 ausfüllende Breite auf. Eine größere Breite weisen die Stützplatten 32 an ihrem anderen freien Ende auf. In ihrem in Längsrichtung mittleren Bereich weisen die Stützplatten 32 jeweils an ihrer einen Seitenkante einen seitlichen Ausschnitt 42 auf, der bis etwa zur Hälfte der Breite der Stützplatten 32 in diese eingreift. An dem der Schwenkachse 40 zugewandten Ende bildet der Ausschnitt 42 eine Gleitkante 44, die parallel zur Schwenkachse 40 und somit senkrecht zur Längserstreckung der Stützplatte 32 verläuft. Die Form der Stützplatten 32 ist am besten in Figur 9 zu erkennen. Die Stützplatten 32.1 und 32.2 an der Oberseite und die Stützplatten 32.3 und 32.4 an der Unterseite sind jeweils um 180° gegeneinandergedreht an den jeweiligen Endstücken 34 bzw. 36 schwenkbar gelagert. Damit greifen die linke Stützplatte 32.1 und die rechte Stützplatte 32.2 an der Oberseite und ebenso die linke Stützplatte 32.3 und die rechte Stützplatte 32.4 an der Unterseite jeweils mit ihren Ausschnitten 42 ineinander. Die linke Stützplatte 32.1 liegt mit ihrem schmalen mittleren Bereich in dem Ausschnitt 42 der rechten Stützplatte 32.2 und liegt auf der Gleitkante 44 dieser rechten Stützplatte 32.2 auf. Umgekehrt greift die rechte Stützplatte 32.2 mit ihrem mittleren Bereich in den Ausschnitt 42 der linken Stützplatte 32.1 und liegt auf deren Gleitkante 44 auf. Entsprechend ist die Anordnung für die unteren Stützplatten 32.3. und 32.4.

Die Spannmittel können auch in dieser zweiten Ausführung teleskopische Spannmittel sein, die ein an dem einen Endstück 34 angeordnetes Außenrohr 16 und einen an dem anderen Endstück 36 angeordneten Innenbolzen 18 aufweisen. Die Spannmittel mit dem Außenrohr 16 und dem Innenbolzen 18 sind in der Mittelachse des Implantats angeordnet. In der zur medianen Sagittalebene senkrechten Horizontalebene sind beiderseits der Spannmittel Führungsmittel angeordnet, die eine gegenseitige Verdrehung der Endstücke 34 und 36 und der an diesen gelagerten Stützplatten 32 um die Mittelachse der Spannmittel verhindern. Die Führungsmittel bestehen in dem Ausführungsbeispiel jeweils aus einem an dem einen Endstück 34 angeordneten Führungsrohr 46 und einer an dem anderen Endstück 36 angeordneten Führungsstange 48. Die Führungsstangen 48 sind jeweils axial gleitend in den Führungsrohren 46 geführt. Die aus den Führungsrohren 46 und den Führungsstangen 48 gebildeten Führungsmittel sind achsparallel zu den Spannmitteln und im gleichen Abstand von diesen angeordnet.

In der in Figur 5 gezeigten Einführstellung des Implantats sind die Endstücke 34 und 36 lateral auseinanderbewegt. Die über ihre Ausschnitte 42 miteinander verschränkten oberen Stützplatten 32.1 und 32.2 und ebenso die über ihre Ausschnitte 42 verschränkten unteren Stützplatten 32.3 und 32.4 liegen in dieser Einführstellung aufeinander auf, so dass die gesamte Höhe des Implantats in vertikaler Richtung gering ist, z.B. 5 bis 7 mm beträgt. Das Implantat kann in dieser Einführstellung unilateral in den Interspinalraum zwischen den Dornfortsätzen zweier aufeinanderfolgender Wirbel eingeführt werden, wie dies oben beschrieben ist. Das Implantat wird im Interspinalraum so positioniert, dass sich das Implantat in lateraler Richtung symmetrisch zur medianen Sagittalebene befindet und die oberen Stützplatten 32.1 und 32.2 dem oberen Dornfortsatz und die unteren Stützplatten 32.3 und 32.4 dem unteren Dornfortsatz zugewandt sind.

Anschließend werden die Spannmittel betätigt, in dem z.B. der Innenbolzen 18 und das Außenrohr 16 teleskopisch zusammengefahren werden. Dadurch werden die Endstücke 34 und 36 in lateraler Richtung zusammengezogen. Dadurch werden auch die oberen Stützplatten 32.1 und 32.2 und entsprechend die unteren Stützplatten 32.3 und 32.4 gegeneinander bewegt. Hierbei gleiten die oberen Stützplatten 32.1 und 32.2 auf der Gleitkante 44 der jeweils anderen Stützplatte 32.2 bzw. 32.1 und ebenso gleiten die unteren Stützplatten 32.3 und 32.4 gegeneinander. Bei dieser Gleitbewegung wird das freie Ende der linken Stützplatte 32.1 von dem rechten Endstück 36 hochgeschwenkt, während umgekehrt das freie Ende der rechten Stützplatte 32.2 von dem linken Endstück 34 hochgeschwenkt wird. Entsprechend werden die freien Enden der unteren Stützplatte 32.3 und 32.4 von den jeweiligen Endstücken 36 bzw. 34 abgeschwenkt. Diese Spreizstellung ist in Figur 6 gezeigt. Durch das Hochspreizen der freien Enden der Stützplatten 32.1 und 32.2 bzw. 32.3 und 32.4 bildet sich zwischen diesen freien Enden der Stützplatten ein mittiger vertiefter Sattel 12. Beim Aufspreizen des Implantats legen sich die hochgeschwenkten Stützplatten 32 an den jeweiligen Dornfortsätzen an, um diese zu distrahieren. Die Dornfortsätze liegen dabei jeweils in dem zwischen den freien Enden der oberen bzw. der unteren Stützplatten 32 gebildeten Sattel 12, so dass das Implantat gegen laterale Verschiebungen formschlüssig an den Dornfortsätzen gehalten ist.

Das Spannen der Spannmittel kann auch in der zweiten Ausführung in derselben Weise erfolgen, wie dies im Zusammenhang mit der ersten Ausführung der Figuren 1 bis 4 beschrieben ist. Dementsprechend kann der Innenbolzen 18 mit einem selbsthemmenden Gewinde in das Außenrohr 16 eingedreht werden.

Eine alternative Ausführung der Spannmittel ist in Figur 10 gezeigt. In dieser Ausführung ist der Innenbolzen 18 mit Rastzähnen 50 an seinem Umfang ausgebildet, die in Rastaufnahmen 52 am Innenumfang des Außenrohres 16 eingreifen. Die Rastzähne 50 und die Rastaufnahmen 52 sind so ausgebildet, dass zum Spannen der Spannmittel der Innenbolzen 18 in das Außenrohr 16 eingedrückt werden kann, während die Rastung ein Herausziehen des Innenbolzens 18 aus dem Außenrohr 16 verhindert. Die Zahnteilung der Rastzähne 50 und der Rastaufnahmen 52 bestimmt dabei die jeweilige Spreizstellung des Implantats. Es ist selbstverständlich, dass diese rastende Ausbildung der Spannmittel auch bei der ersten Ausführung des Implantats verwendet werden kann, die in den Figuren 1 bis 4 gezeigt ist.

Eine Ausführung der Spannmittel, bei welcher diese in aufeinanderfolgenden Spannpositionen einrasten, wie dies bspw. in Figur 10 gezeigt ist, ermöglicht ein Nachspannen des Implantats, z. B. bei Knochenschwund und Knochenresorption durch eine Nachstellkraft, die den Innenbolzen 18 axial in das Außenrohr 16 drückt, um die Spreizstellung um eine Rastposition weiter zu stellen. Diese Nachstellkraft kann selbstverständlich ebenfalls minimalinvasiv mechanisch eingeleitet werden, wie dies bei einer Verstellung mittels eines Gewindes der Fall ist. Ebenso ist es möglich die Nachstellkraft über eine Injektion hydraulisch oder pneumatisch oder induktiv-elektrisch einzuleiten. Ein automatisches Nachstellen kann durch eine integrierte Nachstellfederkraft bewirkt werden. Eine solche Nachstellfederkraft beaufschlagt die Spannmittel in der Spannrichtung, d. h. in der Richtung, in welcher die Stützmittel auseinander gespreizt werden. Solange die Stützmittel an den Dornfortsätzen anliegen, stützen die Dornfortsätze die Stützmittel gegen diese zusätzliche Nachstellfederkraft ab. Geben die Dornfortsätze aufgrund von Knochenschwund nach, so wird die Nachstellfederkraft wirksam und betätigt die Spannmittel in der Weise, dass die Stützmittel weiter aufgespreizt werden und wieder zur Anlage an den Dornfortsätzen kommen. Die Nachstellfederkraft kann bspw. durch Federmittel bewirkt werden, die in die Spannmittel oder bei der Ausführung der Figuren 5 bis 9 gegebenenfalls auch in die Führungsmittel integriert sind. Solche Federmittel können bspw. aus einer Zugfeder bestehen, die in das Außenrohr 16 bzw. die Führungsrohre 46 eingesetzt ist und an dem Innenbolzen 18 bzw. den Führungsstangen 48 angreift und den Innenbolzen 18 in das Außenrohr 16 bzw. die Führungsstangen 48 in die Führungsrohre 46 hinein zieht. Alternativ können Druckfedern vorgesehen sein, die den Innenbolzen 18 in das Außenrohr 16 oder umgekehrt das Außenrohr 16 über den Innenbolzen 18 drücken bzw. entsprechend die Führungsstangen 48 und die Führungsrohre 46 ineinander drücken.

Solche Federmittel können insbesondere auch aus einer Memory-Legierung bestehen. Die Federmittel können dabei beim Einsetzen des Implantats auf einer niedrigen Temperatur gehalten werden, in welcher sie entspannt sind und die Spannmittel nicht beaufschlagen. Erst wenn das Implantat nach dem Einsetzen sich auf die Körpertemperatur des Patienten erwärmt gehen die Memory-Federmittel in den Zustand über, in welchem sie die Nachstellfederkraft bewirken.

### Bezugszeichenliste

- 10: Federblatt
- 12: Sattel
- 14: Zähne
- 16: Außenrohr
- 18: Innenbolzen
- 20: Einschnitte
- 22: Endstück
- 24: Endstück
- 26: Gewinde
- 32: Stützplatten
- 34: Endstück
- 36: Endstück
- 38: Aussparungen
- 40: Schwenkachse
- 41: Bohrungen
- 42: Ausschnitt
- 44: Gleitkante
- 46: Führungsrohre
- 48: Führungsstangen
- 50: Rastzähne
- 52: Rastaufnahmen

## Patentansprüche

1. Dornfortsatz-Implantat mit einem Abstandhalter, der mit zur medianen Sagittalebene im Wesentlichen senkrechter Längsachse zwischen einem oberen und einem anschließenden unteren Dornfortsatz positionierbar ist,
**dadurch gekennzeichnet, dass** der Abstandhalter obere Stützmittel (10.1; 32.1, 32.2) zur Anlage an dem oberen Dornfortsatz und untere Stützmittel (10.2; 32.3, 32.4) zur Anlage an dem unteren Dornfortsatz aufweist, dass die Stützmittel (10; 32) zwischen einer ersten Einführstellung mit geringem Abstand senkrecht zur Längsachse und einer zweiten Spreizstellung mit größerem Abstand senkrecht zur Längsachse aufspreizbar sind, dass die Stützmittel (10; 32) jeweils einen vertieften Sattel (12) aufweisen, mit welchem die Stützmittel (10; 32) im implantiertem Zustand an dem jeweiligen Dornfortsatz anliegen und **dadurch** gegen eine laterale Verschiebung gesichert sind, und dass zwischen den Stützmitteln (10; 32) in Längsrichtung verlaufende Spannmittel angeordnet sind, die an den beiden lateralen Enden der Stützmittel (10; 32) angreifen, um diese aufzuspreizen.

2. Dornfortsatz-Implantat nach Anspruch 1,
**dadurch gekennzeichnet, dass** die Spannmittel in Längsrichtung teleskopisch längenveränderbar sind.

3. Dornfortsatz-Implantat nach Anspruch 2,
**dadurch gekennzeichnet, dass** die Spannmittel ein Außenrohr (16) und einen koaxial in diesem bewegbaren Innenbolzen (18) aufweisen.

4. Dornfortsatz-Implantat nach Anspruch 2 oder 3,
**dadurch gekennzeichnet, dass** die Spannmittel in dem Spreizzustand rastend oder über ein selbsthemmendes Gewinde gehalten sind.

5. Dornfortsatz-Implantat nach einem der vorhergehenden Ansprüche,
**dadurch gekennzeichnet, dass** die Spannmittel Endstücke (22, 24; 34, 36) aufweisen, die an den lateralen Enden der Stützmittel (10; 32) angreifen und zum Aufspreizen der Stützmittel (10; 32) in lateraler Richtung zusammengezogen werden.

6. Dornfortsatz-Implantat nach einem der Ansprüche 1 bis 5,
**dadurch gekennzeichnet, dass** die Stützmittel wenigstens ein Federblatt (10) aufweisen, welches sich in Längsrichtung erstreckt und nach oben bzw. unten auswölbend aufspreizbar ist.

7. Dornfortsatz-Implantat nach Anspruch 6,
**dadurch gekennzeichnet, dass** die Stützmittel ein oberes und ein unteres Federblatt (10.1 und 10.2) aufweisen, die identisch ausgebildet sind, um 180° gegeneinander verdreht angeordnet sind, mit ihren lateralen Enden verschränkt ineinander greifen und durch die Spannmittel konvex nach oben bzw. nach unten ausgewölbt aufspreizbar sind.

8. Dornfortsatz-Implantat nach Anspruch 6 oder 7,
**dadurch gekennzeichnet, dass** der Sattel (12) durch eine konkav gewölbte Vertiefung des Federblattes (10) gebildet ist.

9. Dornfortsatz-Implantat nach einem der Ansprüche 1 bis 5,
**dadurch gekennzeichnet, dass** die Stützmittel durch hochschwenkbare Stützplatten (32) gebildet sind.

10. Dornfortsatz-Implantat nach Anspruch 9,
**dadurch gekennzeichnet, dass** die Spannmittel laterale Endstücke (34, 36) aufweisen, dass die Stützplatten (32) jeweils mit einem Ende an einem der Endstücke (34, 36) schwenkbar gelagert sind, während ihr anderes Ende hochschwenkbar ist.

11. Dornfortsatz-Implantat nach Anspruch 10,
**dadurch gekennzeichnet, dass** die Stützplatten (32) zum Hochschwenken auf einer Gleitkante (44) verschoben werden.

12. Dornfortsatz-Implantat nach Anspruch 11,
**dadurch gekennzeichnet, dass** wenigstens zwei Stützplatten (32) vorgesehen sind, wobei eine Stützplatte (32.1 bzw. 32.3) an dem einen Endstück (34) und die andere Stützplatte (32.2 bzw. 32.4) an dem anderen Endstück (36) schwenkbar gelagert ist und wobei die Stützplatten (32.1 und 32.2 bzw. 32.3 und 32.4) mit seitlichen Ausschnitten (42) verschränkt ineinander greifen und in diesen Ausschnitten (42) jeweils eine Gleitkante (44) ausgebildet ist, an welcher die jeweils andere Stützplatte zum Hochschwenken verschiebbar ist.

13. Dornfortsatz-Implantat nach Anspruch 12,
**dadurch gekennzeichnet, dass** an der Oberseite und an der Unterseite des Implantats jeweils zwei Stützplatten (32.1, 32.2 bzw. 32.3, 32.4) angeordnet sind und diese Stützplatten identische Form aufweisen.

14. Dornfortsatzimplantat nach einem der Ansprüche 5 bis 13,
**dadurch gekennzeichnet, dass** Führungsmittel (46, 48) die Endstücke (22, 24; 34, 36) bei der lateralen Bewegung gegen ein gegenseitiges Verdrehen um die Längsrichtung sichern.

15. Dornfortsatzimplantat nach einem der vorhergehenden Ansprüche,
**dadurch gekennzeichnet, dass**
die Spannmittel durch eine Nachstellkraft in der Spreizrichtung beaufschlagbar sind.

16. Dornfortsatzimplantat nach Anspruch 15,
**dadurch gekennzeichnet, dass** die Nachstellkraft durch in die Spannmittel integrierte Federmittel bewirkt wird.

17. Dornfortsatzimplantat nach Anspruch 16,
**dadurch gekennzeichnet, dass**
die Federmittel aus einer Memory-Legierung bestehen.
